(19) Europäisches Patentamt
European Patent Office
Office européen des brevets

(11) **EP 3 671 757 A1**

(12) **EUROPEAN PATENT APPLICATION**

(43) Date of publication:
**24.06.2020 Bulletin 2020/26**

(51) Int Cl.:
**G16H 40/63** (2018.01)    **G16H 50/30** (2018.01)
**G16H 50/20** (2018.01)    **A61B 5/16** (2006.01)

(21) Application number: **18213285.2**

(22) Date of filing: **18.12.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **DESPENIC, Marija**
**5656 AE Eindhoven (NL)**

• **FERREIRA DOS SANTOS DA FONSECA, Pedro Miguel**
**5656 AE Eindhoven (NL)**
• **AUBERT, Xavier Louis Marie Antoine**
**5656 AE Eindhoven (NL)**

(74) Representative: **de Haan, Poul Erik et al**
**Philips International B.V.**
**Philips Intellectual Property & Standards**
**High Tech Campus 5**
**5656 AE Eindhoven (NL)**

(54) **SYSTEM AND METHOD FOR DETERMINING A LEVEL OF ALERTNESS**

(57)    A system is provided for determining a level of alertness of a subject, using a physiological sensor for providing physiological sensor signals which distinguish between a sleep state and an awake state of the subject. Sleep sessions are identified and their associated falling asleep and waking up times. A single representative midsleep time is obtained from multiple sleep sessions over a 24 hour period, and a circadian phase is estimated using the single representative midsleep time. A level of alertness is then obtained from the circadian phase. This system avoids the need for the use of a default circadian phase when determining a level of alertness. The system can more accurately estimate sleepiness for individuals that have irregular sleep patterns such as shift workers or people with sleep disorders. The single representative midsleep time is also able to take account of multiple sleep sessions, i.e. naps or periods of restless sleep.

FIG. 1

**Description**

FIELD OF THE INVENTION

This invention relates to a system and method for determining a level of alertness, either for analysis of historical information or for future prediction.

BACKGROUND OF THE INVENTION

Individuals can perform better when they are alert. When an individual is sleepy, he/she is more prone to errors which could be life threatening. Sleepiness may be caused by various factors including sleep debt and the time of the day. If sleepiness can be predicted beforehand, its effects can be minimized or prevented, for example by structuring work or taking corrective action (e.g. taking breaks), before or during periods an individual is predicted to be overly sleepy.

A three-process model of alertness known for sleepiness prediction, for example as disclosed in T. Akerstedt and S. Folkard, "The three-process model of alertness and its extension to performance, sleep latency, and sleep length," Chronobiology international, vol. 14, no. 2, pp. 115-123, 1997. It is further discussed in M. Ingre, W. V. Leeuwen, T. Klemets, C. Ullvetter, S. Hough, G. Kecklund, D. Karlsson and T. Åkerstedt, "Validating and extending the three process model of alertness in airline operations," PLoS One, vol. 9, no. 10, 2014.

This model takes into account three main components. The first component is a circadian process that describes diurnal variation in alertness (C). The second component is a homeostatic process that describes the decline of alertness with time awake and its recovery with time asleep (S). The third component is a sleep inertia process that describes the delay after wake up before alertness resumes (W).

Besides the circadian component, there is also an ultradian component (U) that is related and affected by the time of the day. This component is added to the original three-process model to simulate the afternoon dip in alertness. The common assumption is that this dip in alertness is due to the presence of a 12 hour component in alertness. Both circadian (C) and ultradian (U) components use circadian rhythm phase in their calculations.

In the known three-process model of alertness, a default value of circadian rhythm phase, representing a population average, is used. The phase of circadian process C, in particular the acrophase, is for example set to a default value of 16.8 in decimal hours. The original three-process model was based on a wake-up time of 07:45 in the morning. It was assumed that the acrophase of process C occurred almost exactly 9 hours after wake up at 16.8 in decimal hours (16:48).

A problem with this approach is that it assumes that people have regular sleep schedules, which is not the case with shift workers or people with sleep disorders. It would be desirable to be able to take account of the actual circadian rhythm phase of a subject, but this is generally an intrusive process or else an inaccurate one.

There remains a need for a reliable and unobtrusive way to determine and predict a level of alertness of a subject in particular taking into account their actual circadian cycle.

SUMMARY OF THE INVENTION

The invention is defined by the claims.

According to examples in accordance with an aspect of the invention, there is provided a system for determining a level of alertness of a subject, comprising:

an input for receiving physiological sensor signals, from a physiological sensor, which distinguish between a sleep state and an awake state of the subject;
a controller which is adapted, from the physiological sensor signals, to:

identify sleep sessions and derive their associated falling asleep and waking up times;
derive a single representative midsleep time from multiple sleep sessions over a 24 hour period;
estimate a circadian phase using the single representative midsleep time; and
determine a level of alertness from the circadian phase.

The circadian phase is defined with respect to the time at which a specific circadian marker occurs. The marker can be the Core-Body Temperature (CBT) minimum or the Dim-Light Melatonin secretion Onset (DLMO). Normally, for a regularly entrained person having a diurnal way-of-life, the CBT occurs near or a bit later than the middle of the main sleep episode and the DLMO occurs in the early evening near 21:00 or 22:00. The circadian phase may be expressed as a time-interval between the current local time and the time of the chosen marker. For example, waking up 4 hours after the CBT minimum, or going to sleep 2 hours after the DLMO marker. Thus, the circadian phase refers to a particular time period which characterizes the timing of the circadian cycle relative to a physiological marker, i.e. a particular point in that cycle such as a maximum value relative to the marker. The timing of the full cyclic circadian rhythm may be obtained or derived from this information. The term "a circadian phase" should be understood accordingly.

This system avoids the need for the use of a default circadian phase when determining a level of alertness. Instead, the default value is replaced with an estimate of the actual circadian phase (in particular the timing of the peak) which is updated automatically, based on inferring the circadian rhythm phase. Instead of using a value representing an average value of the circadian phase when calculating a circadian component over the whole population, the determination of the level of alertness can be personalized. Thus, using the individual's sleep session durations allows a personalization of the circadian phase estimation, in contrast with solutions that simply use population averages for circadian rhythm phase.

The detection of oscillations in estimated value of the circadian rhythm phase of an individual can also be helpful in determining the presence of a circadian disorder.

The system can also more accurately estimate sleepiness for individuals that have irregular sleep patterns such as shift workers or people with sleep disorders. The single representative midsleep time is also able to take account of multiple sleep sessions, i.e. naps or periods of restless sleep.

The system may be implemented as a wearable device equipped with a sensor. For example, the sensor may comprise a plethysmography (PPG) sensor and/or an acceleration sensor. This provides a non-intrusive and yet objective solution. Other sensor modalities may also be used (such as an EEG) but the aim to enable non-intrusive monitoring.

In this way, the sleep duration may be automatically estimated based on actigraphy analysis of body movements (from the accelerometer) or preferably, from the combination of body movements and autonomic (cardiac and respiratory) activity measured from the PPG signal. As a result of the increased accuracy in the estimation of sleep and wake states, and therefore of sleep duration, the limitation of manual annotations such as in sleep diaries is overcome.

Since the solution is based on a wearable device, and does not require in loco physical examination, sleep lab session, or laboratory tests, it is suitable for free-living conditions.

The system also enables monitoring consecutive periods of 24 hours, which may uncover periods of daytime napping which would otherwise not be taken in to account; the presence, frequency and duration of daytime naps may be indicative of different sleep disorders, including circadian disorders. Existing solutions instead assume that individual has a single main sleep session during the monitoring period.

The controller may be adapted to:

derive respective midsleep times for the sleep sessions;
derive the single representative midsleep time based on a weighted average of the midsleep times of the multiple sleep sessions.

The weighted average is for example weighted according to the duration of the respective sleep session. In this way, longer sleep sessions have more impact on the determination of the midsleep time than shorter naps.

The controller may be adapted to:
determine the circadian phase for a particular day based on the midsleep time for that day and the midsleep time for the following day.

This is possible if analysis is being conducted of historical information. For example, the controller may be adapted to determine a peak of the circadian cycle of a day as the midsleep time for that day (i.e. the night before) plus half the difference in time between the midsleep time for that day and for the following day. This analysis may then be used as the basis for a future prediction of the circadian cycle.

The controller may instead be adapted to:
determine a peak of the circadian cycle for a particular day based on the midsleep time for that day shifted forwards by 12 hours.

This enables real time determination of the circadian phase even with only a single day of monitoring.

The controller may be adapted to determine a level of alertness from the circadian phase based on the three-process model. The invention enables the three-process model to provide more reliable results, because a more accurate determination of the circadian component is made possible.

The controller may be adapted to determine the circadian phase further taking into account the age of the subject.

The system preferably further comprises a display for displaying an alertness indication over time. This may be used to assist a user in structuring their work, or timing sleeping breaks.

The controller may be implemented as part of the wearable physiological sensor or it may be implemented at least partly in a remote device with which the wearable physiological sensor communicates. The sensor may communicate with a portable device of the user such as a phone or tablet (e.g. via Bluetooth), and that portable device may communicate over the internet with a processing resource for performing some or all of the data processing steps.

The invention also provides processing system for determining a level of alertness of a subject based on physiological sensor signals received which distinguish between a sleep state and an awake state of the subject, wherein the processing system is adapted, from the physiological sensor signals, to:

> identify sleep sessions and derive their associated falling asleep and waking up times;
> derive a single representative midsleep time from multiple sleep sessions over a 24 hour period;
> estimate a circadian phase using the single representative midsleep time; and
> determine a level of alertness from the circadian phase.

This defines the processor, which may be local to the sensor or remote from the sensor.

The invention also provides a method for determining a level of alertness of a subject, comprising:

> obtaining physiological sensor signals from a subject which distinguish between a sleep state and an awake state of the subject;
> identifying sleep sessions and derive their associated falling asleep and waking up times;
> deriving a single representative midsleep time from multiple sleep sessions over a 24 hour period;
> estimating a circadian phase using the single representative midsleep time; and
> determining a level of alertness from the circadian phase.

The method may comprise:

> deriving respective midsleep times for the sleep sessions;
> deriving the single representative midsleep time based on a weighted average of the midsleep times of the multiple sleep sessions.

The method may comprise obtaining a weighted average using weightings according to the duration of the respective sleep session.

A peak of the circadian cycle of a day may be determined as the midsleep time for that day plus half the difference in time between the midsleep time for that day and for the following day.

Alternatively, a peak of the circadian cycle for a particular day may be determined based on the midsleep time for that day shifted forwards by 12 hours.

A level of alertness may then be determined from the circadian phase based on the three-process model.

The invention also provides a computer program comprising computer program code means which is adapted, when said program is run on a computer, to implement the method defined above.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

> Fig. 1 shows a system for determining a level of alertness of a subject;
> Fig. 2 shows a method for determining a level of alertness of a subject;
> Fig. 3 shows examples of how different durations of sleep sessions influence the estimation of a single representative midsleep time;
> Fig. 4 shows graphically a first example of how the calculation of circadian rhythm phase is performed;
> Fig. 5 shows graphically a second example of how the calculation of circadian rhythm phase is performed;
> Fig. 6 shows an example of sleepiness prediction results; and
> Fig. 7 illustrates an example of a general architecture of a computer for implementing one or more of the units which implement the controller.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Before describing the approach of the invention, known approaches for determined sleepiness will first be discussed.

As mentioned above, a three-process model of alertness known is known for sleepiness prediction, which takes into account the circadian process (C), the homeostatic process (S) and the sleep inertia process (W).

The components of the original three-process model are calculated in the following way with the default values of the parameters in the parenthesis. The homeostatic sleep component S describes the decline in alertness as a function of wakefulness and recovery in alertness during sleep. During wakefulness, the sleep component S follows:

$$S = (S_a - L)e^{-0.0353\,t_{aw}} + L$$

where L is a lower asymptote of the internal alertness scale (L = 2.4). The parameter $S_a$ ($S_a$= 14) represents the component S at the time of awaking and $t_{aw}$ represents the time since awake (in decimal hours).

During sleep, there are three different functions describing the homeostatic process, but which are not taken into account when predicting sleepiness. The model also includes a sleep inertia process W that initially reduces alertness at the time of waking up ($W_{init}$ = -5.72) with an exponential recovery ($W_{decay}$ = -1.51) as a function of time being awake ($t_{aw}$):

$$W = W_{init}e^{W_{decay}t_{aw}}$$

There are two components which are related to the time of the day ($t_{od}$ - in decimal hours). Process C has a period of 24 h with a default circadian phase (p = 16.8) and amplitude (M = 2.5):

$$C = Mcos(\frac{\pi}{12}(t_{od} - p)) \qquad (1)$$

Process U has a period of 12 hours with amplitude ($U_a$ = 0.5) and mesor ($U_m$ = -0.5):

$$U = U_m + U_acos(\frac{\pi}{6}(t_{od} - p - 3)) \qquad (2)$$

For sleepiness prediction, the model components are added together to produce a combined alertness score that can be used to predict the ratings on the Karolinska Sleepiness Scale (KSS). The Karolinska Sleepiness Scale, which describes a subjective, self-perception of alertness and sleepiness, contains values in range from 1 to 9 with the following definitions: 1: extremely alert; 2: very alert; 3: alert; 4: rather alert; 5: neither alert nor sleepy; 6: some signs of sleepiness; 7: sleepy, but no effort to keep awake; 8: sleepy, some effort to stay away; 9: very sleepy, great effort to keep awake, fighting sleep.

The sleepiness is predicted using a linear transformation function with one constant (a = 10.6) and one coefficient (b = -0.6):

$$KSS = a + b(S + C + W + U) \qquad (3)$$

Two components of the three-process model of alertness - circadian component (C) (Equation 1) and ultradian component (U) (Equation 2), use an estimation of the circadian rhythm phase p. In the original three-process model of alertness, this parameter has a default value of 16.8 in decimal hours. This value represents the average over population that does not take into account the shift-workers or people with sleep disorders. This can have a significant impact on alertness prediction.

Circadian rhythms are endogenous rhythms with a periodicity of approximately 24 hours. These rhythms are synchronized to the physical environment by social and work schedules by various photic and nonphotic stimuli. Circadian rhythm represents a repeating pattern based on the natural progression of night and day.

The biological circadian clock in humans for example gives the following typical patterns throughout a 24 hour period, for someone who rises early in the morning, has lunch around noon and sleeps at 23:00:

| | |
|---|---|
| 02:00 | deepest sleep |
| 04:30 | lowest body temperature |
| 06:45 | sharpest blood pressure rise |
| 07:30 | melatonin secretion stops |
| 10:00 | highest alertness |
| 14:30 | best coordination |
| 15:30 | fastest reaction time |
| 17:00 | greatest cardio-vascular efficiency and muscle strength |
| 18:30 | highest blood pressure |
| 19:00 | highest body temperature |
| 21:00 | melatonin secretion stops |

Circadian rhythms allow organisms to anticipate and prepare for precise and regular environmental changes. Rhythmicity appears to be as important in regulating and coordinating internal metabolic processes, as in coordinating with the environment.

Either disruption of the endogenous circadian control mechanism or misalignment between the internal clock time and the 24-hour external environment can result in circadian rhythm disorders with adverse consequences in sleep and many other aspects of human health, including metabolism dysfunction, cognitive impairment, cardiovascular abnormalities and genito-urinary dysfunctions. Disruption in rhythms in the longer term is believed to have significant adverse health consequences in peripheral organs outside the brain, in particular in the development or exacerbation of cardiovascular diseases. Also, misalignment of the circadian timing system with the external environment (e.g. light-dark cycle) might play a role in the development of metabolic disorders (e.g. obesity and diabetes).

Circadian Rhythm Sleep Disorders (CRSDs) arise from a chronic or recurrent pattern of sleep and wake disturbance that is due to dysfunction of the circadian clock system, or misalignment between the timing of the endogenous circadian rhythm and externally imposed social and work cycles that result in clinically significant functional impairments. CRSDs can be categorized according to their postulated underlying mechanisms:

1. The endogenous circadian clock itself is altered:

    1.1 Delayed sleep phase disorder,
    1.2 Advanced sleep phase disorder,
    1.3 Irregular sleep wake rhythm, and
    1.4 Free-running disorder

2. The external environment and/or social circumstances are altered relative to the endogenous circadian clock:

    2.1 Jet lag and
    2.2 Shift work disorder.

These disorders are characterized by different patterns of sleep time over the course of a series of 24 hour periods.

Delayed sleep phase disorder (DSPD) is a sleep disorder in which there is a stable delay of the major sleep episode relative to the required sleep/wake clock time. This delayed pattern leads to chronic symptoms of insomnia and excessive sleepiness associated with impairment in daytime functioning.

Advanced Sleep Phase Disorder (ASPD) is a sleep disorder in which there is a stable advance of the major sleep period, characterized by habitual and involuntary sleep onset and wake-up times that are several hours earlier than the desired or conventional clock time. Patients with ASPD typically present with complaints of sleepiness in the late afternoon or early evening and difficulty maintaining asleep in the early morning hours.

Non-24 Hour Sleep Wake Disorder (N24HSWD, formerly known as free-running rhythm disorder) is characterized by a chronic or recurring pattern of sleep and wake times that are not stably entrained to the 24-hour environmental cycle. There is typically a predictable drift over weeks (usually to later and later times) of sleep onset and wake times. Patients with N24HSWD present symptoms similar to insomnia, with difficulty waking up in the morning, excessive daytime sleepiness and inability to meet their social and occupational obligations.

Irregular sleep-wake rhythm Disorder (ISWRD) is characterized by temporal disorganization of the circadian sleep-wake rhythm, resulting in multiple short sleep and wake bouts occurring throughout the 24-hour cycle. Patients with ISWR or their caregivers typically report chronic symptoms of difficulty maintaining sleep during the night and excessive daytime sleepiness.

Shift work Disorder (SWD) occurs when work hours are scheduled during the habitual sleep times leading to chronic complaints similar to insomnia and excessive daytime sleepiness and resulting impairment of function that are temporarily associated with the unconventional work schedules. In humans, shift-work that favors irregular eating times is associated with altered insulin sensitivity and higher body mass. Shift-work also leads to increased metabolic risks for cardio-metabolic syndrome, hypertension, and inflammation. Other symptoms of SWD include chronic fatigue, malaise, mood disorder, gastrointestinal problems and decreased libido. Risk of alcohol and substance abuse is increased, as is the risk of weight gain, hypertension, cardiovascular disease and breast and endometrial cancer. In addition to the medical co-morbidities, SWD is associated with significant loss of productivity, increased health care utilization and increased risk for personal and public safety.

Jet-lag disorder results from travel across several time zones and subsequent misalignment of the internal circadian clock and the destination's local time. Regarding jet-lag, in general, it is more difficult to adapt to eastward travel. For eastward travelers, the symptoms at the destination include difficulty falling asleep, excessive daytime sleepiness and decreased daytime performance especially in the morning. For westward travelers, falling asleep is less problematic than maintaining sleep, and early evening sleepiness and decreased performance are especially troublesome. Other common associated symptoms of jet lag include altered appetite and gastrointestinal function, general malaise, fatigue and mood disturbance.

The timing of the phase of circadian rhythms can be an indicator of existing CRSDs. Knowing the phase of circadian rhythm can help in early detection of particular CRSD.

The classic phase markers for measuring the timing of circadian rhythm are:

    1. Core body temperature minimum
    2. Plasma level of cortisol
    3. Melatonin secretion by the pineal gland (measured from plasma, saliva or urinary samples)

Temperature control (thermoregulation) is part of a homeostatic mechanism that keeps the organism at optimum operating temperature, as it affects the rate of chemical reactions. Temperatures cycle regularly up and down through the day, as controlled by the person's circadian rhythm. The core body temperature of an individual tends to have the lowest value in the second half of the sleep cycle; the lowest point, called the nadir, is one of the primary markers for circadian rhythms. Though variation is great among normal chronotypes (individuals without a circadian misalignment or disorder), the average adult human temperature reaches its minimum at about 4 a.m. or 5 a.m., about two hours before habitual wake time.

Cortisol has one of the most distinct circadian rhythms in human physiology. It is regulated by the central clock located in the suprachiasmatic nucleus of the hypothalamus. It has been suggested that cortisol acts as a secondary messenger between central and peripheral clocks, hence its importance in the synchronization of body circadian rhythms. Normal individuals have very low or undetectable cortisol levels at midnight that build up overnight to peak first thing in the morning. Cortisol levels then decline slowly throughout the day.

Melatonin is a hormone produced by pinealocytes in the pineal gland. Melatonin helps regulate the circadian rhythm. It is naturally synthesized from the amino acid tryptophan. Production of melatonin by the pineal gland is under the influence of the suprachiasmatic nucleus of the hypothalamus (SCN) which receives information from the retina about the daily pattern of light and darkness. The human body produces its own melatonin starting two hours before the bedtime, provided that lighting is dim. This natural action is known as 'dim light melatonin onset' (DLMO) and helps keeping the body on a regular sleep-wake pattern. The time of onset of melatonin secretion in dim light, measured in plasma or saliva, is frequently used as a marker for the phase of the circadian rhythm. The amount of melatonin secretion during various time of the day, for a normal person, goes through a 24 hour cycle. The secretion of melatonin peaks in the middle of the night and gradually falls during the second half of the night. DLMO is considered the best test available - a 'gold standard', for measuring the phase time and the existence of circadian rhythm disorders. Taking the DLMO test is very helpful for discovering and understanding disturbances in the human biological clock. More importantly, DLMO has significant correlation with midpoint of sleep (midsleep time) which can therefore be used to estimate the phase of the circadian rhythm.

The relationships between DLMOS and sleep timing (onset, midpoint and wake time) are discussed in:

    S. K. Martin and C. I. Eastman, "Sleep logs of young adults with self-selected sleep times predict the dim light melatonin onset," Chronobiology International, vol. 19, no. 4, pp. 695-707, 2002;
    S. J. Crowley, C. Acebo, G. Fallone and M. A. Carskadon, "Estimating Dim Light Melatonin Onset (DLMO) Phase in Adolescents Using Summer or School-Year Sleep/Wake Schedules," Sleep, vol. 29, no. 12, pp. 1632-1641, 2006;

and

J. Terman, M. Terman, E. Lo and T. Cooper, "Circadian time of morning light administration and therapeutic response in winter depression," Arch Gen Psychiatry, vol. 58, pp. 69-75, 2001.

In the known three-process model of alertness, a default value of circadian rhythm phase, representing a population average, is used. The phase of circadian process C is for example set to a default value of 16.8 in decimal hours.

As mentioned above, a problem with this approach is that it assumes that people have regular sleep schedules, which is not the case with shift workers or people with sleep disorders. It would be desirable to be able to take account of the actual circadian rhythm phase of a subject.

Current methods for the estimation of the circadian rhythm phase include different analyses. Rectal temperature examination is the traditional gold standard measurement used to estimate core body temperature. For temperature studies, subjects must remain awake but calm and semi-reclined in near darkness while their rectal temperatures are taken continuously. Plasma and salivary cortisol are usually measured every 2 hours. Measurements of salivary cortisol are used as a surrogate for measurement in serum/plasma. For plasma cortisol measurements, an intravenous catheter can be used. Salivary samples for cortisol are obtained by placing a swab in the mouth and chewing for about 60 seconds to simulate salivation.

The DLMO test has to be performed at a sleep lab and usually takes place from 8pm until 3am. During the test, patients are asked to stay in dim lighting and every hour samples of saliva, in the form of chewed cotton balls, are collected. Afterwards, the melatonin concentration of the samples is measured. During the test, the patient cannot eat or drink 30 minutes before each sample is collected. These types of measurements are intrusive, uncomfortable and are not feasible in free-living conditions.

Given the inadequacy of these methods to assess the circadian rhythm of individuals, in particular over longer periods of days, or weeks, the clinical diagnosis of circadian rhythm sleep disorders usually resorts to sleep history and physical examination using sleep logs and/or actigraphy for more than 7 days. Sleep logs can be obtained manually or in a sleep lab. Self-assessed manual annotations consisting of sleep diaries are less intrusive than measurements of core body temperature or the collection of cortisol or melatonin samples, but are highly inaccurate due to rounded sleep and wake times, forgotten naps, lack of annotations of awakenings during a night and sleep state misperception.

Another approach commonly used is the Morningness-Eveningness Questionnaire (MEQ) or the Munich Chrono Type Questionnaire (MCTQ). These questionnaires are also based on approximations and suffer from the same inaccuracies as sleep diaries.

MEQ-based questionnaires reportedly yield plausible results, yet in terms of "real-world" behavior, they do not explicitly model free days and workdays separately nor do they ask for actual sleep times or exposure to outdoor light. However, individual sleep times show wide variations between work and free days (except for extreme early chronotypes) and depend on a subject's exposure to daylight. MCTQ questionnaire quantitatively assess the timing of sleep within the 24 hour day (sleep phase) using simple questions. Calculation of the midsleep time on free days (MSF) for circadian rhythm phase estimation is then performed based on questions concerning sleep onset and awakening time on days on which there are no work or social obligations. MSF is the mid-point between these two times. To compensate for the differences existing between work and free days, MSF is then corrected as:

$$ \mathrm{MSF}_{sc} = \mathrm{MSF} - 0.5 * (\mathrm{SD_F} - (5 * \mathrm{SD_W} + 2 * \mathrm{SD_F})/7) $$

where $\mathrm{SD_W}$ represents sleep duration on work days and $\mathrm{SD_F}$ represents sleep duration on free days. Term $(5 * \mathrm{SD_W} + 2 * \mathrm{SD_F})/7$ is the average weekly sleep duration. Although this approach takes into account differences between work and free days for midsleep time calculation, it suffers from several limitations:

1. it is based on (subject) questionnaire input by the individual being examined, and
2. it assumes that there is only one (main) sleep session during the day. Calculating the circadian phase based on midsleep time estimated this way does not take into account possible naps that can indicate existence of irregular sleep-wake rhythm disorder or shift-work disorder.

The invention provides a system for determining a level of alertness of a subject, using a physiological sensor (such as a wearable sensor) for providing physiological sensor signals which distinguish between a sleep state and an awake state of the subject. Sleep sessions are identified and their associated falling asleep and waking up times. A single representative midsleep time is obtained from multiple sleep sessions over a 24 hour period, and a circadian phase is estimated using the single representative midsleep time. A level of alertness is then obtained from the circadian phase. This system avoids the need for the use of a default circadian phase when determining a level of alertness. The system can more accurately estimate sleepiness for individuals that have irregular sleep patterns such as shift workers or people with sleep disorders. The single representative midsleep time is also able to take account of multiple sleep sessions, i.e. naps or periods of restless sleep.

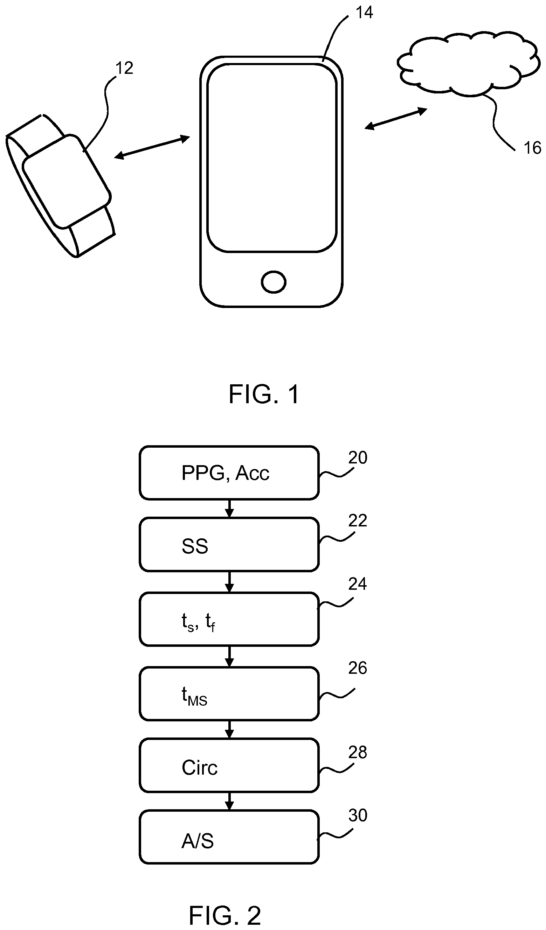

Fig. 1 shows a system 10 for determining a level of alertness of a subject. It comprises a wearable physiological sensor 12 for providing physiological sensor signals which distinguish between a sleep state and an awake state of the subject. Note that non-wearable sensors may also be used such as bed sensors.

The sensor may be a wrist-worn PPG sensor, accelerometers for measuring body movement, an EEG sensor integrated into a headband, or any combination of these. Accelerometers enable actigraphy analysis of body movements, wherein absence of movement for a period is indicative of sleep, and PPG sensors enable cardiac and respiratory activity to be measured.

Since the solution is based on a wearable device, and does not require in loco physical examination, sleep lab session, or laboratory tests, it is suitable for free-living conditions. The sensor also enables the duration of each sleep episode to be determined. These sleep episodes may be a main sleep session during the night, or daytime naps

The sensor 12 may have all the processing capability built in. However, Fig. 1 instead shows that the sensor 12 communicates using a short range wireless communication protocol (e.g. Bluetooth) with a mobile telephone 14 on which is stored an suitable app, and the mobile telephone connects over the internet to a remote data processing function 16. The processing functions in all three units shown may be considered to constitute a controller. Thus, it will be understood that the controller as defined in this application may be located in one hardware device, or it may be distributed between multiple devices.

The controller analyzes the physiological sensor signals to identify sleep sessions and derive their associated falling asleep and waking up times.

The invention in particular enables multiple sleep sessions to be identified over a 24 hour period, such as naps or periods of disturbed sleep. Each sleep session has an associated midsleep time, and from these times a single representative midsleep time is deriver relating to the 24 hour period.

A circadian phase may then be estimated using the single representative midsleep time.

This circadian phase is for example a waveform over time. One particular value, such as timing of the peak, may be of interest for a subsequent derivation of a level of alertness from the circadian phase.

This system avoids the need for the use of a default circadian phase when determining a level of alertness. Instead, the default value is replaced with an estimate of the actual circadian phase (in particular the timing of the peak) which is updated automatically, based on inferring the circadian rhythm phase. This provides a personalization of the circadian phase estimation, in contrast with solutions that simply use population averages for circadian rhythm phase.

Fig. 2 shows the method for determining a level of alertness of a subject, as implemented by the controller.

In step 20, physiological sensor signals are obtained from a subject. These signals distinguish between a sleep state and an awake state of the subject. This step is thus performed by the sensor device.

In step 22, sleep states (SS) are identified. These sleep states include at least an awake state and an asleep state. However, further sleep states may be identified.

For example, different sleep stages are associated with different brain activity characteristics, which may be sensed by an EEG monitor. In particular, the brain activity of subject may be associated with rapid eye movement (REM) sleep, non-rapid eye movement (non-REM) sleep. Sleep stages may include one or more of non-REM stage N1, stage N2, or stage N3 sleep, REM sleep. N1 corresponds to a light sleep state and N3 corresponds to a deep sleep state. Non-REM stage N3 or stage N2 sleep may be slow wave activity (SWA, i.e., deep) sleep.

Thus, in a refinement, different sleep stages may also be taken account rather than only falling asleep and waking up times.

Note that other techniques are available which do not measure brainwave activity but measure cardiac and respiratory activity to automatically distinguish different sleep stages. Thus, sleep stage analysis may also be conducted using wearable monitors. Sleep stages may be also affected by (misalignments in) the circadian rhythm, and can help identify and also quantify such misalignments.

In step 24, sleep sessions are identified and their associated start (falling asleep) times $t_s$ and finish (waking up) times ($t_f$).

In step 26 a single representative midsleep time $T_{MS}$ is derived from the multiple sleep sessions over a 24 hour period.

In step 28, a circadian phase (Circ) is estimated using the single representative midsleep time and a level of alertness / sleepiness A/S) is estimated in step 30 from the circadian phase.

These steps, apart from the initial data collection by the sensor, are performed by the controller, as explained above, which may in one place or distributed between different devices.

As explained above, the 'dim light melatonin onset' (DLMO) is considered the best available measurement - 'gold standard' -, for estimating the circadian phase. DLMO has significant correlation with midpoint of sleep (a midpoint between sleep onset and wake up time). Therefore, based on the midsleep time, the phase of the circadian rhythm can be estimated.

In a case when a person had several sleep sessions, a midsleep time for a specific day is estimated as weighted average of all midsleep times in the previous 24 hours (prior to awakening) where the weighting factor is the duration of the specific sleep session:

$$t_{MS} = \frac{\sum_{i=1}^{N} t_{ms}^i \Delta t_s^i}{\sum_{i=1}^{N} \Delta t_s^i} \tag{4}$$

where $t_{ms}^i$ and $\Delta t_s^i$ are the midsleep time and duration of a session i, respectively, and N is the number of sleep sessions in the previous 24 hours prior to awakening.

In this way, respective midsleep times are derived for the multiple sleep sessions and then a single representative midsleep time is based on a weighted average of the midsleep times of the multiple sleep sessions.

The midsleep time is thus based on all sleep sessions, without assuming that there is only one, main session during a day. It allows personalization of an alertness model taking into account naps and fragmented rest episodes. Also, it allows to obtain further insight in potential circadian rhythm sleep disorders by looking at the oscillations in the estimated values for circadian rhythm phase.

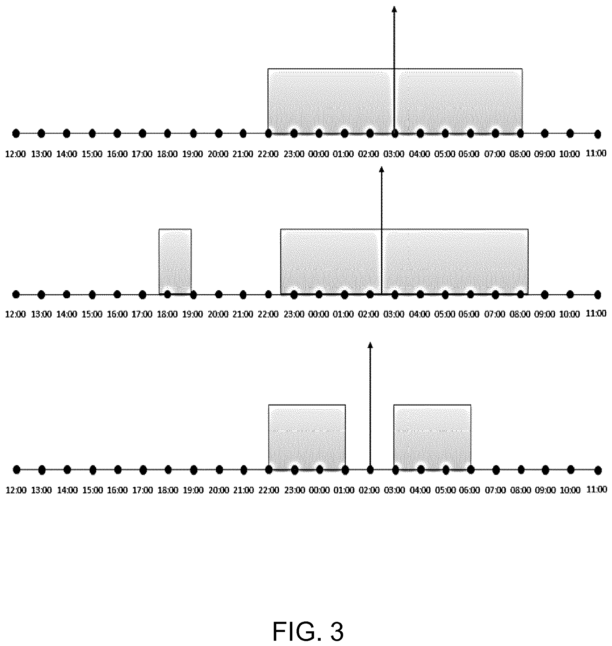

Examples of how different durations of sleep sessions influence the estimation of the single representative midsleep time are presented in Fig. 3.

The plots show the sleep times, and the single arrow in each plot shows the timing of the single representative midsleep time.

The top plot relates to a single main sleep session. In this case, no weighted average is needed, and the midsleep time is obtained in a known manner.

The middle plot shows one main sleep session during a night and a nap during a day. The bottom plot shows two separate sleep sessions during a night with equal duration.

Based on the single representative midsleep time, the circadian rhythm phase can be inferred.

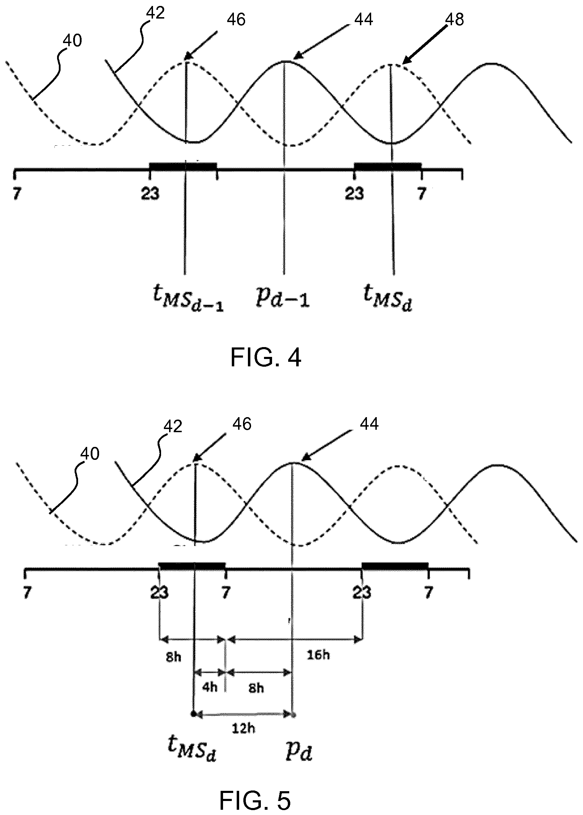

Fig. 4 shows graphically how the calculation of circadian rhythm phase is performed. Plot 40 line represents the melatonin secretion cycle, while plot 42 represents the circadian rhythm cycle. The circadian rhythm phase for a day d - 1 can be estimated based on two consecutive midsleep times as:

$$p_{d-1} = t_{MS_{d-1}} + \frac{t_{MS_d} - t_{MS_{d-1}}}{2} \tag{5}$$

where $t_{MS_d}$ is the midsleep time for a day d, and $t_{MS_{d-1}}$ is the midsleep time for a previous day (d - 1).

The circadian phase is thus determined for a particular day based on the midsleep time for that day and the midsleep time for the following day. In particular a peak 44 of the circadian phase of a day is calculated as the midsleep time 46 for that day plus half the difference in time between the midsleep time 46 for that day and the midsleep time 48 for the following day.

This calculation is only possible on a day d (i.e. the next day). If this is the first day of calculation, or the calculation of circadian rhythm phase needs to be performed in online fashion (without the midsleep time of the next day), the following approximation can be taken:

$$p_d = t_{MS_d} + 12h \tag{6}$$

In this case, a peak 44 of the circadian phase for a particular day is based on the midsleep time 46 for that day shifted forwards by 12 hours.

This calculation is shown in Fig. 5. It assumes that a person has approximately 8 hours of sleep. To take into account the differences between midsleep times during free and workdays, for calculation of circadian rhythm phase, 7 consecutive days are preferably taken into account.

Furthermore, to compensate for possible outliers, a median value of estimated circadian rhythm phase over the past 7 days may be taken for use in the final estimation.

If the difference between midsleep times is more than 7 days, because the user did not wear the device, the calculation of circadian rhythm phase is reset and started all over again, since no particular sleep behavior is assumed when wearable is not worn.

The three process model (as discussed above) may then be used (in step 30 of Fig. 2) to personalize the sleepiness prediction based on the circadian rhythm phase estimated as explained above.

Both circadian (C) and ultradian (U) components in the original three-process model use a default value of 16.8 (in decimal hours) of circadian rhythm phase in their calculations. The default value in Equation (1) and (2) can be replaced by the automatically evaluated circadian rhythm phase from Equation (5) or (6) based on the single representative midsleep time obtained by Equation (4).

In this way the model can more accurately estimate sleepiness for individuals that have irregular sleep patterns such as shift workers or people with sleep disorders.

For sleepiness prediction, the model components are added together to produce a combined alertness score that can be used to predict the ratings on the 1-9 Karolinska Sleepiness Scale (KSS) explained above, using a linear transformation function as shown above Equation (3).

In a preferred embodiment, the sleepiness prediction algorithm calculations are performed remotely. As the result of the calculations, the predicted KSS values for a period after wake up (e.g. 16 hours since awakening) are sent back for visualization to the user, for an example on an app on a smartphone.

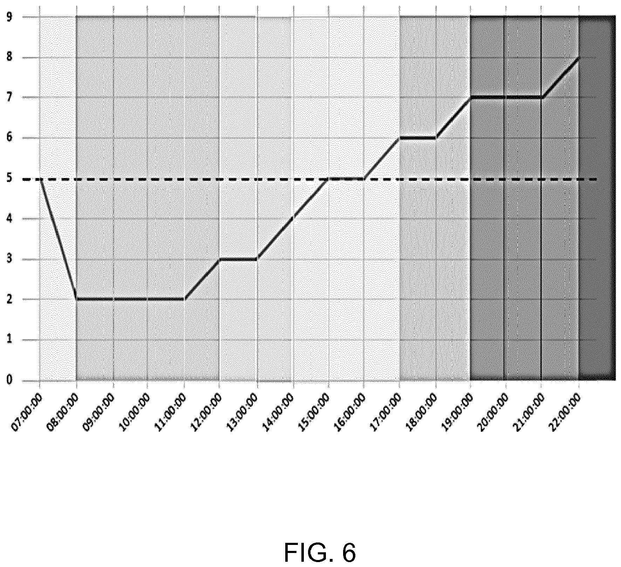

An example of sleepiness prediction results is presented in Fig. 6, where the y-axis is the KSS rating.

Additional parameters may be used to further optimize the phase parameter used for sleepiness prediction. Age, and sensor-derived sleep parameters (duration, fragmentation) can all be used to further personalize other parts of the model. For example if a subject had a number of nights with exceptionally short sleep duration, a penalty can be used in the calculation of the S process or even of the sleepiness based on the three processes (Equation (3) above implements this).

Similarly, age can be used to adjust the different constants used in the three-process model, or to establish normative values for sleep duration and sleep fragmentation which can be in turn used for comparison with actual sleep measures and can further adjust the sleepiness prediction (e.g. by penalizing it in case of increased fragmentation or decreased duration, or by reducing it in case of a particularly long and restful night of sleep).

The system can also more accurately estimate sleepiness for individuals that have irregular sleep patterns such as shift workers or people with sleep disorders. The single representative midsleep time is also able to take account of multiple sleep sessions, i.e. naps or periods of restless sleep.

The analysis performed by the system of the invention allows sleeping session information and circadian phase information to be displayed for a previous period for example of multiple days, as well as for predicting the timing of a next circadian phase and hence predicting an alertness level, for example for the next 24 hour period or for a number of future days. The predicted alert level may be displayed on a KSS scale. The system enables these predictions and analyses to be obtained based as a minimum only on sensing the onset of sleep and awakening from sleep, for long sleep sessions as well as naps and different sleep periods of fragmented sleep. Thus, non-invasive sensing is possible.

Predicting sleepiness using the method and system described above could help avoiding these issues by structuring the work or taking corrective action (e.g. taking breaks), during the time an individual is predicted to be sleepy. Shift workers are particularly prone to sleepiness due to irregular sleeping patterns. This solution would allow workers and their managers to get insight in individual alertness state of their workers, allowing them to design shift work based on it and avoid potentially harmful situations.

The controller may be implemented as part of the wearable physiological sensor or it may be implemented at least partly in a remote device with which the wearable physiological sensor communicates. The sensor may communicate with a portable device of the user such as a phone or tablet (e.g. via Bluetooth, over a USB cable, or using NFC, WiFi, etc.), and that portable device may communicate over the internet with a processing resource for performing some or all of the data processing steps.

If all processing is instead performed by the wearable device, it does not need to communicate the raw data for off-device processing, instead performing all computations on an on-board processor and finally displaying the results to the user on an included display, or sending (e.g. via Bluetooth or other communication mechanism) the results of the processing to an external display (e.g. on a paired smartphone).

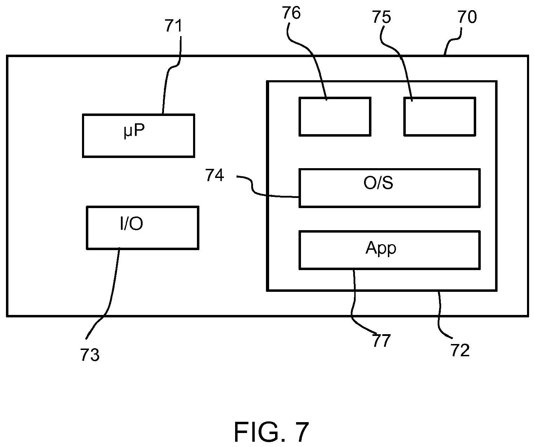

Fig. 7 illustrates an example of a general architecture of a computer 70 for implementing one or more of the units which implement the controller described above.

The computer 70 includes, but is not limited to, PCs, workstations, laptops, PDAs, palm devices, servers, storages, and the like. Generally, in terms of hardware architecture, the computer 70 may include one or more processors 71, memory 72, and one or more I/O devices 73 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 71 is a hardware device for executing software that can be stored in the memory 72. The processor 71 can be virtually any custom made or commercially available processor, a central processing unit (CPU), a digital signal processor (DSP), or an auxiliary processor among several processors associated with the computer 70, and the processor 71 may be a semiconductor based microprocessor (in the form of a microchip) or a microprocessor.

The memory 72 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 72 may incorporate electronic, magnetic, optical, and/or other types of storage media. Note that the memory 72 can have a distributed architecture, where various components are situated remote from one another, but can be accessed by the processor 71.

The software in the memory 72 may include one or more separate programs, each of which comprises an ordered listing of executable instructions for implementing logical functions. The software in the memory 72 includes a suitable operating system (O/S) 74, compiler 75, source code 76, and one or more applications 77 in accordance with exemplary embodiments.

The application 77 comprises numerous functional components such as computational units, logic, functional units, processes, operations, virtual entities, and/or modules.

The operating system 74 controls the execution of computer programs, and provides scheduling, input-output control, file and data management, memory management, and communication control and related services.

Application 77 may be a source program, executable program (object code), script, or any other entity comprising a set of instructions to be performed. When a source program, then the program is usually translated via a compiler (such as the compiler 75), assembler, interpreter, or the like, which may or may not be included within the memory 72, so as to operate properly in connection with the operating system 74. Furthermore, the application 77 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, and the like.

The I/O devices 73 may include input devices such as, for example but not limited to, a mouse, keyboard, scanner, microphone, camera, etc. Furthermore, the I/O devices 73 may also include output devices, for example but not limited to a printer, display, etc. Finally, the I/O devices 73 may further include devices that communicate both inputs and outputs, for instance but not limited to, a network interface controller (NIC) or modulator/demodulator (for accessing remote devices, other files, devices, systems, or a network), a radio frequency (RF) or other transceiver, a telephonic interface, a bridge, a router, etc. The I/O devices 73 also include components for communicating over various networks, such as the Internet or intranet.

When the computer 70 is in operation, the processor 71 is configured to execute software stored within the memory 72, to communicate data to and from the memory 72, and to generally control operations of the computer 70 pursuant to the software. The application 77 and the operating system 74 are read, in whole or in part, by the processor 71, perhaps buffered within the processor 71, and then executed.

When the application 77 is implemented in software it should be noted that the application 77 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

**Claims**

1. A system for determining a level of alertness of a subject, comprising:

   an input for receiving physiological sensor signals, from a physiological sensor (12), which distinguish between a sleep state and an awake state of the subject;
   a controller (14,16) which is adapted, from the physiological sensor signals, to:

identify sleep sessions and derive their associated falling asleep and waking up times;
derive a single representative midsleep time from multiple sleep sessions over a 24 hour period;
estimate a circadian phase using the single representative midsleep time; and
determine a level of alertness from the circadian phase.

2. A system as claimed in claim 1, wherein the controller is adapted to:

   derive respective midsleep times for the sleep sessions;
   derive the single representative midsleep time based on a weighted average of the midsleep times of the multiple sleep sessions, for example weighted according to the duration of the respective sleep session.

3. A system as claimed in any one of claims 1 to 2, wherein the controller is adapted to:

   determine the circadian phase for a particular day based on the midsleep time for that day and the midsleep time for the following day.

4. A system as claimed in claim 3, wherein the controller is adapted to determine a peak of the circadian cycle of a day as the midsleep time for that day plus half the difference in time between the midsleep time for that day and for the following day.

5. A system as claimed in any one of claims 1 to 2, wherein the controller is adapted to:

   determine a peak of the circadian cycle for a particular day based on the midsleep time for that day shifted forwards by 12 hours.

6. A system as claimed in any one of claims 1 to 5, wherein the controller is adapted to determine a level of alertness from the circadian phase based on the three-process model.

7. A system as claimed in any one of claims 1 to 6, wherein the controller is adapted to determine the circadian phase further taking into account the age of the subject.

8. A system as claimed in any one of claims 1 to 7, wherein:

   the controller is implemented as part of the physiological sensor; or
   the controller is implemented at least partly in a remote device with which the wearable physiological sensor communicates.

9. A system as claimed in any one of claims 1 to 8, further comprising the physiological sensor, and wherein the physiological sensor comprises a plethysmography sensor and/or an acceleration sensor.

10. A processing system for determining a level of alertness of a subject based on physiological sensor signals received which distinguish between a sleep state and an awake state of the subject, wherein the processing system is adapted, from the physiological sensor signals, to:

    identify sleep sessions and derive their associated falling asleep and waking up times;
    derive a single representative midsleep time from multiple sleep sessions over a 24 hour period;
    estimate a circadian phase using the single representative midsleep time; and
    determine a level of alertness from the circadian phase.

11. A method for determining a level of alertness of a subject, comprising:

    (20) obtaining physiological sensor signals from a subject which distinguish between a sleep state and an awake state of the subject;
    (22) identifying sleep sessions and (24) derive their associated falling asleep and waking up times;
    (26) deriving a single representative midsleep time from multiple sleep sessions over a 24 hour period;
    (28) estimating a circadian phase using the single representative midsleep time; and
    (30) determining a level of alertness from the circadian phase.

**12.** A method as claimed in claim 11, comprising:

deriving respective midsleep times for the sleep sessions;
deriving the single representative midsleep time based on a weighted average of the midsleep times of the multiple sleep sessions, for example using weightings according to the duration of the respective sleep session.

**13.** A method as claimed in any one of claims 10 to 12, comprising:

determining a peak of the circadian cycle of a day as the midsleep time for that day plus half the difference in time between the midsleep time for that day and for the following day; or
determining a peak of the circadian cycle for a particular day based on the midsleep time for that day shifted forwards by 12 hours.

**14.** A method as claimed in any one of claims 10 to 13, comprising determining a level of alertness from the circadian phase based on the three-process model.

**15.** A computer program comprising computer program code means which is adapted, when said program is run on a computer, to implement the method of any one of claims 10 to 14.

FIG. 1

FIG. 2

12:00 13:00 14:00 15:00 16:00 17:00 18:00 19:00 20:00 21:00 22:00 23:00 00:00 01:00 02:00 03:00 04:00 05:00 06:00 07:00 08:00 09:00 10:00 11:00

12:00 13:00 14:00 15:00 16:00 17:00 18:00 19:00 20:00 21:00 22:00 23:00 00:00 01:00 02:00 03:00 04:00 05:00 06:00 07:00 08:00 09:00 10:00 11:00

12:00 13:00 14:00 15:00 16:00 17:00 18:00 19:00 20:00 21:00 22:00 23:00 00:00 01:00 02:00 03:00 04:00 05:00 06:00 07:00 08:00 09:00 10:00 11:00

FIG. 3

FIG. 4

FIG. 5

FIG. 6

71

76　75　70

μP

74

O/S

I/O

App

73

77　72

FIG. 7

Europäisches
Patentamt

European
Patent Office

Office européen
des brevets

## EUROPEAN SEARCH REPORT

Application Number

EP 18 21 3285

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2017/238868 A1 (KENYON MATT [US] ET AL) 24 August 2017 (2017-08-24) * summary; paragraphs [0076] - [0086]; figures 1,3,8 * | 1-15 | INV. G16H40/63 G16H50/30 G16H50/20 |
| A | US 2014/296332 A1 (DRESSMAN MARLENE MICHELLE [US] ET AL) 2 October 2014 (2014-10-02) * paragraphs [0172] - [0196], [0204], [0210] * | 1-15 | ADD. A61B5/16 |
| X | US 2017/132946 A1 (KINNUNEN HANNU OLAVI [FI] ET AL) 11 May 2017 (2017-05-11) * summary, paragraphs 75,88-89 in particular; paragraphs [0054] - [0057], [0059], [0060], [0071], [0072], [0074], [0122], [0127], [0128], [0179] * | 1-15 | |
| A | US 2012/191425 A1 (MOTT CHRISTOPHER [CA] ET AL) 26 July 2012 (2012-07-26) * paragraphs [0058] - [0060], [0080] * | 1-15 | TECHNICAL FIELDS SEARCHED (IPC) G16H A61B |

The present search report has been drawn up for all claims

| Place of search | Date of completion of the search | Examiner |
|---|---|---|
| Munich | 12 June 2019 | Huber, Alexander |

EPO FORM 1503 03.82 (P04C01)

## ANNEX TO THE EUROPEAN SEARCH REPORT
## ON EUROPEAN PATENT APPLICATION NO.

EP 18 21 3285

This annex lists the patent family members relating to the patent documents cited in the above-mentioned European search report.
The members are as contained in the European Patent Office EDP file on
The European Patent Office is in no way liable for these particulars which are merely given for the purpose of information.

12-06-2019

| Patent document cited in search report | Publication date | Patent family member(s) | Publication date |
|---|---|---|---|
| US 2017238868 A1 | 24-08-2017 | CA 3014812 A1 | 24-08-2017 |
| | | CN 108697391 A | 23-10-2018 |
| | | EP 3416557 A1 | 26-12-2018 |
| | | JP 2019510550 A | 18-04-2019 |
| | | KR 20180111926 A | 11-10-2018 |
| | | US 2017238868 A1 | 24-08-2017 |
| | | WO 2017143179 A1 | 24-08-2017 |
| US 2014296332 A1 | 02-10-2014 | AU 2013211878 A1 | 21-08-2014 |
| | | AU 2013211880 A1 | 21-08-2014 |
| | | AU 2016204178 A1 | 14-07-2016 |
| | | AU 2016204217 A1 | 14-07-2016 |
| | | AU 2018201302 A1 | 15-03-2018 |
| | | AU 2018201909 A1 | 12-04-2018 |
| | | CA 2861108 A1 | 01-08-2013 |
| | | CA 2861111 A1 | 01-08-2013 |
| | | CL 2014001992 A1 | 13-03-2015 |
| | | CL 2014001993 A1 | 13-03-2015 |
| | | CN 104379135 A | 25-02-2015 |
| | | CN 104519877 A | 15-04-2015 |
| | | CO 7131353 A2 | 01-12-2014 |
| | | CO 7131366 A2 | 01-12-2014 |
| | | DK 2806863 T3 | 30-10-2017 |
| | | EP 2806863 A2 | 03-12-2014 |
| | | EP 2806864 A2 | 03-12-2014 |
| | | EP 3300728 A2 | 04-04-2018 |
| | | ES 2646197 T3 | 12-12-2017 |
| | | HR P20171600 T1 | 15-12-2017 |
| | | HU E034876 T2 | 28-03-2018 |
| | | JP 6267260 B2 | 24-01-2018 |
| | | JP 2015508157 A | 16-03-2015 |
| | | JP 2015509106 A | 26-03-2015 |
| | | JP 2016172737 A | 29-09-2016 |
| | | JP 2017039731 A | 23-02-2017 |
| | | JP 2018080181 A | 24-05-2018 |
| | | JP 2019038822 A | 14-03-2019 |
| | | KR 20140116927 A | 06-10-2014 |
| | | KR 20140117537 A | 07-10-2014 |
| | | KR 20170058463 A | 26-05-2017 |
| | | KR 20180100450 A | 10-09-2018 |
| | | KR 20190006598 A | 18-01-2019 |
| | | PT 2806863 T | 09-11-2017 |
| | | RU 2014134552 A | 20-03-2016 |
| | | RU 2014134555 A | 20-03-2016 |
| | | SI 2806863 T1 | 29-12-2017 |
| | | US RE46604 E | 14-11-2017 |

EPO FORM P0459

For more details about this annex : see Official Journal of the European Patent Office, No. 12/82

## ANNEX TO THE EUROPEAN SEARCH REPORT
## ON EUROPEAN PATENT APPLICATION NO.

EP 18 21 3285

This annex lists the patent family members relating to the patent documents cited in the above-mentioned European search report.
The members are as contained in the European Patent Office EDP file on
The European Patent Office is in no way liable for these particulars which are merely given for the purpose of information.

12-06-2019

| Patent document cited in search report | Publication date | Patent family member(s) | Publication date |
|---|---|---|---|
|  |  | US 2013197076 A1 | 01-08-2013 |
|  |  | US 2014296330 A1 | 02-10-2014 |
|  |  | US 2014296331 A1 | 02-10-2014 |
|  |  | US 2014296332 A1 | 02-10-2014 |
|  |  | US 2014357710 A1 | 04-12-2014 |
|  |  | US 2015216837 A1 | 06-08-2015 |
|  |  | US 2017087123 A1 | 30-03-2017 |
|  |  | US 2017100366 A1 | 13-04-2017 |
|  |  | US 2018071245 A1 | 15-03-2018 |
|  |  | US 2019070144 A1 | 07-03-2019 |
|  |  | WO 2013112949 A2 | 01-08-2013 |
|  |  | WO 2013112951 A2 | 01-08-2013 |
| US 2017132946 A1 | 11-05-2017 | NONE |  |
| US 2012191425 A1 | 26-07-2012 | US 2012191425 A1 | 26-07-2012 |
|  |  | WO 2009052633 A1 | 30-04-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **T. AKERSTEDT ; S. FOLKARD.** The three-process model of alertness and its extension to performance, sleep latency, and sleep length. *Chronobiology international,* 1997, vol. 14 (2), 115-123
- **M. INGRE ; W. V. LEEUWEN ; T. KLEMETS ; C. ULLVETTER ; S. HOUGH ; G. KECKLUND ; D. KARLSSON ; T. ÅKERSTEDT.** Validating and extending the three process model of alertness in airline operations. *PLoS One,* 2014, vol. 9 (10
- **K. MARTIN ; C. I. EASTMAN.** Sleep logs of young adults with self-selected sleep times predict the dim light melatonin onset. *Chronobiology International,* 2002, vol. 19 (4), 695-707
- **J. CROWLEY ; C. ACEBO ; G. FALLONE ; M. A. CARSKADON.** Estimating Dim Light Melatonin Onset (DLMO) Phase in Adolescents Using Summer or School-Year Sleep/Wake Schedules. *Sleep,* 2006, vol. 29 (12), 1632-1641
- **TERMAN ; M. TERMAN ; E. LO ; T. COOPER.** Circadian time of morning light administration and therapeutic response in winter depression. *Arch Gen Psychiatry,* 2001, vol. 58, 69-75